# EUROPEAN PATENT APPLICATION

(11) **EP 3 266 458 A1**
(43) Date of publication of application: **10.01.2018**
(21) Application number: 16177896.4
(22) Date of filing: 05.07.2016
(51) Int. Cl.: A61K 31/5575, A61K 9/00, A61K 47/00, A61P 15/10

(54) **METHOD AND COMPOSITION AND KIT FOR TREATING ERECTILE DYSFUNCTION**

(71) Applicant: Fagron B.V., 3062 ME Rotterdam (NL)
(72) Inventor: Padilla, Rafael, São Paulo (BR); Neder, Geraldino, São Paulo (BR)
(74) Representative: Nederlandsch Octrooibureau

(57) **Abstract**

The present invention concerns the field of a pharmaceutical composition for treatment of male erectile dysfunction. The present invention also concerns a method for providing said pharmaceutical composition and an applicator containing said pharmaceutical formulation. Furthermore, the invention concerns the use of the pharmaceutical composition and the applicator with the pharmaceutical composition in the treatment of male erectile dysfunction. Finally, the invention concerns a kit comprising an applicator containing the pharmaceutical composition for treatment of male erectile dysfunction, a cooling container and instructions.

## Description

### TECHNICAL FIELD OF THE INVENTION

The present invention concerns the field of pharmaceutical compositions for treatment of male erectile dysfunction. The present invention also concerns a method for providing said pharmaceutical composition and an applicator containing said pharmaceutical formulation. Furthermore, the invention concerns the pharmaceutical composition and the applicator with the pharmaceutical composition for use in the treatment of male erectile dysfunction. Finally, the invention concerns a kit comprising an applicator containing a pharmaceutical composition for treatment of male erectile dysfunction, a cooling container and instructions.

### BACKGROUND OF THE INVENTION

Approximate 150 million men suffer from erectile dysfunction (ED), worldwide. Oral therapies for treating ED, *e.g.* tablets comprising phosphodiesterase type 5 inhibitors, have been developed and have proven to be an effective and efficient first line of therapy. However, a sub-population of men suffering from ED remains, that is excluded from the use of such oral therapies, due to their medical conditions or medical history. In addition, such oral therapies are shown ineffective when taken by a sub-population of men suffering from ED, though not responding to an, e.g., phosphodiesterase type 5 inhibitor. Therefore, alternative first-line therapies for ED patients excluded from taking oral ED medication at forehand, were searched for. In addition, additive second-line therapies were searched, *e.g.* for use in combination therapy, for ED patients responding insufficiently to available oral therapies.

*Alprostadil* (IUPAC name: *7-[(1R,2R,3R)-3-hydroxy-2-[(E,3S)-3-hydroxyoct-1-enyl]-5-oxocyclopentyl]heptanoic acid),* the synthetic variant of natural vasoactive prostaglandin E1 (PGE1), is heavily investigated and tested for its efficacy in treatment of ED. The method of action of PGE1 such as alprostadil is independent of the nitric oxide - cGMP pathway, which is the target for phosphodiesterase type 5 inhibitors used for oral therapies. Instead, alprostadil activates adenylate cyclase, resulting in cAMP accumulation, with the effect of corporal smooth muscle relaxation in the penis, allowing an increase in blood flow to the penis facilitating an erection to build up. Therefore, alprostadil is selected as an attractive alternative for effective ED therapy for ED patients not responsive to phosphodiesterase type 5 inhibitors.

US5,919,474 describes a medicated pellet comprising *e.g.* 500 microgram PGE1 for delivery in the urethra of the penis. The pellet is to be delivered in the urethra with a device, at a depth of at least 3 to 7 cm in the urethra. The device comprises a transurethral inserter having a shaft sized to fit the urethra and comprising the PGE1 pellet. Inserting the stem of an applicator for about 3 to 7 cm in the urethra prior to PGE1 deposition and releasing a pellet in the urethra causes adverse events such as pain in 33% to 50% of the patients and bleeding in about 4% of patients.

In Leungwattanakij et al. (Urologic Clinics of North America, 2001), Moisidis et al. (Current Opinion in Urology, 2016) and Costa & Potempa (Drugs, 2012), alprostadil for intraurethral delivery and a method for intraurethral delivery of alprostadil, have been described. A pellet comprising 125, 250, 500 or 1,000 microgram alprostadil formulated in polyethylene glycol is contained in the tip of an applicator with a hollow stem 3.2 cm in length. The tip is 3 or 6 mm in length. For administering the alprostadil pellet, the stem, or sheath, is fully inserted into the distal urethra immediately after micturition, and the pellet is deposited. The alprostadil pellet then dissolved in urine remains in the urethra. Inserting the stem in the urethra may induce pain. Also the deposition of a pellet inside the urethra may cause inconvenience. Moreover, patients experience hesitation to insert an applicator into their penis and deposit a solid composition inside the urethra.

Indeed, a 32% discontinuation rate was reported in patients treated with an applicator comprising PGE1 and a sheath to be inserted in the urethra for delivery of the PGE1, due to adverse effects of the medication [Saleh *et al.,* 2015].

In EP1255552B, a semi-solid PGE1 composition for treating ED is disclosed further comprising a skin penetration enhancer selected from the group consisting of an alkyl-2-(N,N-disubstituted amino)-alkanoate and an (N,N-disubstituted amino)-alkanol alkanoate. The semi-solid PGE1 composition is administered in the fossa navicularis of the penis of the ED patient. The semi-solid PGE1 composition is to be administered using an applicator with a tip, which tip is to be applied over a distance of 0.5 to 2 cm into the fossa navicularis. As for inserting the longer sheath of 3 to 7 cm, as outlined above, also inserting a sheath of 0.5 to 2 cm in length bears the risk for pain and fear to apply for the ED patient. Inserting the applicator through the meatus may induce pain. Patients experience hesitation to insert an applicator into their penis and inside the fossa navicularis.

Alternative to the semi-solid PGE1 compositions for invasive application in the urethra or in the fossa navicularis by deposition using a sheath to be inserted inside the penis through the meatus, PGE1 compositions for external application have been developed [Becher, 2004; Padma-Nathan & Yeager, 2006; Moncada & Cuzin, 2015; Moisidis *et al.,* 2016; international application WO2004022064A1

Alprostadil in a composition comprising skin permeation enhancer selected from an alkyl N,N-dimethylaminoacetate and an N,N-dimethylalkanol ester of an alkanoic acid, has been described. The composition is topically administered at the meatus of the glans penis. The composition is topically applied to the meatus by releasing droplets of the alprostadil composition above the meatus. Alprostadil topical doses were 50, 100, 200 and 300 microgram [Becher, 2004]. Alternatively, doses of 100, 200 or 300 microgram alprostadil formulated as a topical composition were administered dropwise onto the meatus of the glans penis using a dispenser [Padma-Nathan & Yeager, 2006]. A modest improvement in erectile function was reported in a population of men with mild to severe ED. Yet another alprostadil formulation for dropwise application was applied in a drop wise action to the meatus with the help of a dispenser [Moisidis *et al.,* 2016; Moncada & Cuzin, 2015]. Alprostadil doses were 200 or 300 microgram. The alprostadil formulation comprised absorption enhancer dodecyl-2-n,n-dimethylaminopropionate-HCl. Ease-of-use of administration of these alprostadil formulations is hampered by the difficulty for the ED patient to direct the droplets of the composition precisely onto the meatus and the glans penis. Waste and leakage of alprostadil composition is a concern, since the fluidic composition is easily spoilt.

WO2004022064A1 describes a composition for the treatment of ED, comprising alprostadil and penetration enhancer dodecyl 2-(N,N-dimethylamino)-propionate HCl. The composition is to be applied in a dropwise manner to the fossa navicularis by applying drops of the formulation into the glans meatus. A dosage of the composition comprises 100 to 400 microgram alprostadil. Viscosity of the fluidic composition is between 5,000 to 20,000 mPa.s. Again, efficacy of administering the alprostadil formulation is hampered by the difficulty for the ED patient to direct the droplets of the composition precisely into the glans meatus. Furthermore, waste of the compositions and leakage of alprostadil composition from the penis is a concern, since the fluidic composition is easily spoilt.

Alternative to the route of administration based on providing PGE1 composition into the meatus or onto the meatus of the glans penis by applying the PGE1 composition in a dropwise manner from a certain distance above the glans penis, administering PGE1 composition using an applicator to be pressed onto the meatus has been described in US20080287879A1. The applicator comprises two syringes, with a first syringe comprising lyophilized alprostadil and a second syringe comprising a carrier gel having a pH above 9. Before use of the alprostadil composition, it has to be prepared by vigorous mixing of the lyophilized alprostadil and the gel for 20-30 times. Before use, a penile adapter made of polyethylene is placed at one end of a syringe, after mixing. The adapter fits within the meatus of the glans penis and a seal between the adaptor and the meatus is created. Herewith, supplying alprostadil onto the penis is prevented. In a study group with men suffering from ED, 40.4% had erections that were sufficient for penetration after administration of the alprostadil composition. Apart from the inconvenience of the multi-component applicator by not being a ready-to-use device when an erection is desired, *i.e*. mixing and assembling is required, the adaptor is made of rigid material in order to provide a seal with the meatus. Pressing the adaptor to the meatus for the purpose to fit the adapter within the meatus can induce an unpleasant feeling, *e.g.* pain. Furthermore, the size of the adaptor hampers appropriate sight at the meatus while positioning the adaptor within the meatus. This introduces the risk for improper positioning of the adaptor, and subsequently deposition of the alprostadil composition outside the aimed location since no seal between the adaptor and the meatus will be formed.

Since all of the currently known alprostadil compositions and applicators for administering such compositions have their drawbacks, there is a strong desire for an improved method of administration of a PGE1 composition. All of the aforementioned combinations of routes of administration by applying several different applicators and varying PGE1 compositions have their drawbacks related to poor efficacy of the therapy, or adverse events or inconvenience upon application of a device and composition, or difficulties in applying the PGE1 composition at the selected location, or combinations thereof. The desired improved method of administration of an PGE1 composition therefore should provide for an easy and convenient way to apply a PGE1 composition, resulting in efficacious treatment of ED with less side effects.

### SUMMARY OF THE INVENTION

A first aspect of the present invention relates to a pharmaceutical composition comprising vasoactive prostaglandin and a pharmaceutically acceptable carrier, said carrier comprising:
a. lecithin;
b. isopropyl palmitate; and
c. urea,
wherein said carrier further comprises either isopropyl myristate or a polaxamer, preferably Polaxamer 407, and
wherein the carrier is formulated as a cream with a viscosity at 25°C of between 25.000 to 200.000 mPa.s.

A second aspect of the present invention relates to a method for preparing a pharmaceutical composition of the invention, comprising the steps of:
a. providing a pharmaceutically acceptable carrier wherein the carrier is formulated as a cream with a viscosity at 25°C of between 25.000 to 200.000 mPa.s, preferably between 40.000 to 150.000 mPa.s, more preferably between 50.000 to 100.000 mPa.s, said carrier comprising:
   i. lecithin;
   ii. isopropyl palmitate; and
   iii. urea,
      and said carrier further comprising either isopropyl myristate or a polaxamer, preferably Polaxamer 407;
b. providing a vasoactive prostaglandin, preferably alprostadil, and a solvent;
c. dissolving said prostaglandin in the solvent; and
d. mixing the dissolved vasoactive prostaglandin with said carrier,
wherein the pH of the carrier is between 4.0 and 6.0, preferably between 4.5 and 5.5, and the pharmaceutical composition is refrigerated to between about 2°C to about 8°C

A third aspect of the present invention relates to a pharmaceutical composition of the invention or obtainable by the method of the invention for use in the treatment of erectile dysfunction.

A fourth aspect of the present invention relates to a ready-to-use applicator comprising a pharmaceutical composition of the invention or obtainable by the method of the invention, wherein the applicator comprises a flexible tip, preferably a flexible silicon tip, with an approximate diameter of between 0.30 cm and 1.00 cm, preferably between 0.50 cm and 0.65 cm, more preferably between 0.55 cm and 0.59 cm, and wherein the applicator contains between 1 and 250 unit doses of the vasoactive prostaglandin, preferably between 2 and 150 unit doses, more preferably between 3 and 100 unit doses.

One embodiment of the invention is the pharmaceutical composition of the invention, wherein said pharmaceutical composition is administered with the ready-to-use applicator of the invention.

A fifth aspect of the present invention relates to a kit comprising:
a. a ready-to-use applicator comprising the pharmaceutical composition of the invention or obtainable by the method of the invention;
b. a container for storage of the applicator and for maintaining the temperature of the pharmaceutical composition in the applicator at or below a predetermined temperature for a predetermined period of time; and
c. instructions regarding storage and use of the applicator and use of the pharmaceutical composition.

A sixth aspect of the present invention is the use of the pharmaceutical composition of the invention or obtainable by the method of the invention for the manufacture of a medicament.

One embodiment of the invention is the use of the pharmaceutical composition of the invention or obtainable by the method of the invention for the manufacture of a medicament, wherein the medicament is for the treatment of erectile dysfunction.

### DESCRIPTION OF THE FIGURES

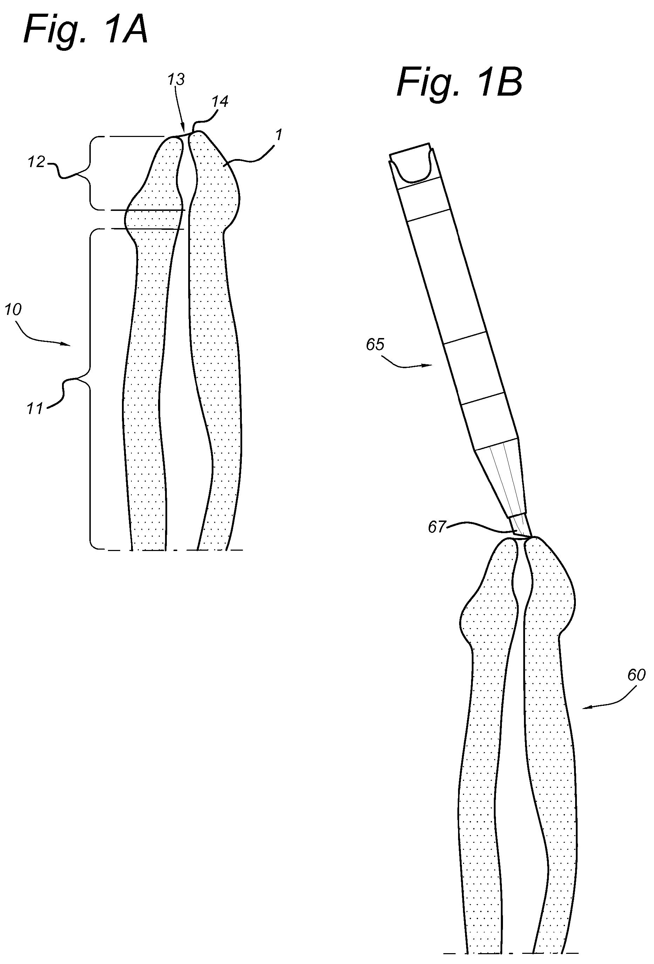

**Figure 1****.** Schematic drawings showing a longitudinal cross-section of a flaccid human penis (10, 60). In (A), the urethra is indicated with (11), the fossa navicularis with (12), the meatus of the glans penis with (13) and with (14) the rim of the meatus at the tip of the glans penis (1).

In (B), the flexible tip (67) of applicator (65) is loosely positioned at the rim of the meatus (14) of the glans (1) of the flaccid penis (10). The distal opening of the flexible tip is positioned above the meatus (13) of the glans penis (1), such that the pharmaceutical composition contained in the applicator is administrable into the fossa navicularis (12) and at the rim of the meatus.

### DEFINITIONS

The term *vasoactive* has its conventional meaning and in the present invention refers to the effect on blood vessels resulting in increasing or decreasing blood pressure.

The term *cream* has its conventional meaning and refers to a semi-solid emulsion of oil and water.

The term *erectile dysfunction,* abbreviated to *ED,* has its conventional meaning and refers to the persistent inability to achieve or maintain penile erection sufficient for satisfactory sexual performance.

The term *rim of the meatus* has its conventional meaning and refers to the external part of the glans penis, projected around the meatus of the glans penis.

The term *meatus of the glans penis,* or *meatus* in short, has its regular scientific meaning and throughout the specification refers to the external urethral orifice, *i.e*. the opening of the fossa navicularis.

The *term fossa navicularis* has its regular scientific meaning and here refers to the part of the male urethra located at the glans penis portion. The fossa navicularis is lined by stratified squamous, non-keratinizing epithelium.

The term *flaccid* has its regular scientific meaning and here refers to a penis insufficiently erected for developing sexual activity in humans.

The term *flexible* has its conventional meaning and in the invention refers to the ability of a material to deform elastically when gently brought in contact with a part of the human body and return to its original shape when released from the body.

The term *Carbomer,* or *Carbopol,* has its conventional meaning and in the invention, Carbomer or Carbopol refers to high-molecular cross-linked polymers of acrylic acid. The preferred carbomer for application in the pharmaceutical composition of the invention is for example *Carbomer 980 NF Polymer,* which is a high-molecular polymer of acrylic acid comprising cross-links with ethers of pentaerythritol, known in the art (see Datasheet for Carbopol 980 NF Polymer, of 16 January 2015, Lubrizol (BE)).

The term *ready-to-use* has its conventional meaning and here refers to the applicability of the pharmaceutical composition of the invention and the applicator comprising the pharmaceutical composition of the invention, without the necessity of any preparative measures before use.

The term *polaxamer,* or *poloxamer,* has its conventional meaning and here refers to a non-ionic triblock copolymer consisting of a central hydrophobic block or chain of polyoxypropylene (poly(propylene oxide)) flanked by two hydrophilic blocks or chains of polyoxyethylene (poly(ethylene oxide)), known in the art. For polaxamers, the approximate lengths of the two polyethylene glycol blocks is between 2 and 130 repeat units while the approximate length of the propylene glycol block is between 15 and 67 repeat units. A preferred polaxamer for application in the pharmaceutical composition of the invention is *Polaxamer 407* (See the Datasheet for Polaxamer 407, sold under the tradename *Kolliphor P 407* by BASF, dated 24 April 2013). For Polaxamer 407, the approximate lengths of the two polyethylene glycol blocks is 101 repeat units while the approximate length of the propylene glycol block is 56 repeat units.

The term *Transcutol P* has its conventional meaning and refers to a purified diethylene glycol monoethyl ether with chemical formula C₂H₅O-CH₂-CH₂-O-CH₂-CH₂-OH and a molecular weight of 134,17 g/mol, with IUPAC-name 2-(2-ethoxyethoxy)ethanol (see: Handling sheet TRANSCUTOL P, Gattefossé, Saint Priest, France, dated 29 June 2010; Technical Data sheet TRANSCUTOL P, specification number 3260 / 4, dated 22 September 2008, Gattefossé, Saint Priest, France). The 2-(2-ethoxyethoxy)ethanol is also traded under the tradename *Carbitol.*

The term *AMP-95* has its conventional meaning and refers to the 95% by weight product 2-amino-2-methyl-1-propanol solution containing 5% added water (See: ANGUS Chemie GmbH Technical Data Sheet AMP-95, CAS Registry No. 124-68-5, ANGUS Chemie GmbH, Ibbenbüren, DE).

The *term Lipopeg 39-S* has its conventional meaning and refers to a polyethylene glycol stearate wherein the polyethylene glycol is polyethylene glycol 40 (See: Safety Data Sheet, Ixom Operations Pty Ltd (Bronson & Jacobs division), Villawood, AU, dated 20 October 2015 - Version 3).

### ABBREVIATIONS

API, active pharmaceutical ingredient; cAMP, cyclic adenosine monophosphate; cGMP, cyclic guanosine monophosphate; ED, erectile dysfunction; EDTA, ethylenediaminetetraacetic acid; IUPAC, International Union of Pure and Applied Chemistry; mPA.s, milli-Pascal-second; PEG, polyethylene glycol; PGE1, prostaglandin E1; qs, quantity sufficient; w/v, weight per volume.

### DETAILED DESCRIPTION OF THE INVENTION

A first aspect of the present invention relates to a pharmaceutical composition comprising vasoactive prostaglandin and a pharmaceutically acceptable carrier, said carrier comprising:
a. lecithin;
b. isopropyl palmitate; and
c. urea,
wherein said carrier further comprises either isopropyl myristate or a polaxamer, preferably Polaxamer 407, and wherein the carrier is formulated as a cream with a viscosity at 25°C of between 25.000 to 200.000 mPa.s.

According to the invention, preferably, the viscosity of the carrier cream at 25°C is between 40.000 mPa.s and 200.000 mPa.s, preferably between 40.000 mPa.s and 150.000 mPa.s, more preferably, between 50.000 mPa.s and 100.000 mPa.s. One embodiment of the invention is the pharmaceutical composition of the invention, wherein the viscosity of the carrier cream at 25°C is between 50.000 and 75.000 mPa.s, preferably about 60.000 mPa.s. One embodiment of the invention is the pharmaceutical composition of the invention, wherein the viscosity of the pharmaceutical composition at 20,5°C is between 50.000 and 90.000 mPa.s, preferably between 60.000 mPa.s and 80.000 mPa.s, more preferably about 70.000 mPa.s. One embodiment of the invention is the pharmaceutical composition of the invention, wherein the viscosity of the pharmaceutical composition at 4°C is between 550.000 and 850.000 mPa.s, preferably between 600.000 mPa.s and 750.000 mPa.s, more preferably about 675.000 mPa.s. According to the invention, said viscosities are determined according to standard methods known in the art, and is for example carried out in a rotational viscosimeter (Fungilab; model Visco Basic Plus), equipped with a Heldal Unit (used for determining viscosity of substances that do not flow).

The combination of lecithin, isopropyl palmitate and urea either with isopropyl myristate, or with a polaxamer, preferably with Polaxamer 407, comprised by the pharmaceutically acceptable carrier in the pharmaceutical composition of the invention, enhances efficient and efficacious skin penetration of active pharmaceutical ingredients such as a vasoactive prostaglandin. Excellent percutaneous absorption performance by a cream comprising isopropyl myristate, lecithin, isopropyl palmitate and urea has for example been demonstrated for steroids [H.C. Polonini *et al.,* 2014].

The inventors found that combining in the pharmaceutical composition of the invention
1. the combination of lecithin, isopropyl palmitate and urea either with isopropyl myristate, or with a polaxamer, preferably with Polaxamer 407, facilitating enhanced skin penetration of the vasoactive prostaglandin;
   and
2. the selected viscosity of the cream, *i.e*. the pharmaceutically acceptable carrier according to the invention,
together, provide a formulation that is optimal for transferring through epithelium a vasoactive prostaglandin to its side of action inside the penis, *i.e*. the corpora cavernosa.

The combination of lecithin, isopropyl palmitate and urea with either isopropyl myristate, or with a polaxamer, preferably Polaxamer 407, facilitates fast uptake of the vasoactive prostaglandin at the side of application, within minutes. The viscosity of the cream allows for convenient application of a dosage in a unit dose of the pharmaceutical composition at the desired part of the human body, whereas at the same time the viscosity of the cream prevents leakage and thereby departure from the site of application. Furthermore, the viscosity of the cream allows for rapid and convenient lubricating the area of application of the pharmaceutical composition. Thus, the pharmaceutical composition of the invention is easily dosed, remains at the site of application for a sufficient period of time to allow for uptake of the vasoactive prostaglandin through the epithelium at the side of application, and uptake of the vasoactive prostaglandin through the epithelial layer is conveniently supported by rubbing the cream in the area of application.

Herewith, the pharmaceutical composition of the invention is particularly suitable for aiding the migration of a vasoactive prostaglandin from its side of administration to its side inside the penis were it exerts its activity, *i.e*. the corpora cavernosa, in the context of a male subject suffering from ED.

One embodiment of the invention is a pharmaceutical composition according to the invention, wherein the vasoactive prostaglandin is selected from natural prostaglandin E1, synthetic prostaglandin, preferably synthetic prostaglandin E1, more preferably alprostadil, a functional derivative of any of these prostaglandins, or any combination of these prostaglandins and/or a functional derivative thereof.

Synthetic PGE1 suitable for use in the treatment of ED is known in the art. For example, synthetic PGE1 *misoprostol* is described for its applicability in an ED treatment regime. One embodiment of the invention is a pharmaceutical composition according to the invention, wherein the vasoactive prostaglandin is alprostadil. Other natural prostaglandins and other synthetic counterparts are equally suitable for application in the pharmaceutical composition of the invention, if administering these alternative or additive compounds to ED patients is beneficial to the patients with regard to treatment of the ED. However, alprostadil is a preferred PGE1 for application in the pharmaceutical composition according to the invention, related to alprostadil's established safety profile and its established efficacy when administered subcutaneously to patients, *i.e*. by intra-cavernosal injection.

One embodiment of the invention is a pharmaceutical composition according to the invention, wherein the composition of the invention comprises between 0.3 and 1.0% by weight vasoactive prostaglandin, preferably about 0.5% by weight. Preferably, the vasoactive prostaglandin is alprostadil. Dosing of the vasoactive prostaglandin is of utmost importance when the pharmaceutical composition of the invention is used in the treatment of ED. According to the invention, an erection should be established fast and to an extent sufficient for desired sexual activity, upon administration of the pharmaceutical composition. The inventors now found for example that a dosage of the pharmaceutical composition of the invention comprising 200 microgram alprostadil was efficient and sufficient in inducing a satisfactory erection in ED patients. One dosage of the pharmaceutical composition of the invention should be available in a dosage form allowing for easy, fast and reliable administration, according to the invention. Thus, the amount of pharmaceutical composition comprising a single dosage, *i.e*. a unit dose, should be enough for convenient and reliably dispensing the pharmaceutical composition at the side of application. In one embodiment of the invention, the amounts of alprostadil to be administered according to the invention are dosages of 20 microliter, 40 microliter and 60 microliter, comprising 100 or 200 or 300 microgram alprostadil, respectively. In yet another embodiment of the invention, a unit dose of 100 microgram alprostadil is comprised in 20 microliter of the pharmaceutical composition. The pharmaceutical composition of the invention comprising 0.5% by weight alprostadil proved to allow for convenient, fast and reliable administration to the ED patient.

An important benefit of the pharmaceutical composition of the invention is the fact that the composition is ready-to-use according to the invention. Before application of the pharmaceutical composition of the invention, dissolving of any constituent is not required, neither mixing of the composition. This is highly beneficial to the application of the pharmaceutical composition of the invention, since it strongly contributes to the easy and fast use when an erection is desired by the ED patient using the pharmaceutical composition of the invention. The time to sexual activity for which the erection is desired is not delayed by any necessary procedural steps before the pharmaceutical composition of the invention is administered.

As said, the inventors now found that in particular the compounds lecithin, isopropyl palmitate and urea, either combined with isopropyl myristate, or combined with a polaxamer, preferably combined with Polaxamer 407, which enhance penetration of vasoactive prostaglandin, preferably alprostadil, through the skin, of the pharmaceutical composition of the invention, contributes to the convenient ease and speed at which an erection is established once the composition is administered to an ED patient.

One embodiment of the invention is the pharmaceutical composition according to the invention, wherein the pharmaceutically acceptable carrier comprised by the pharmaceutical composition comprises between 10% and 14% by weight isopropyl myristate, between 2.5% and 3.5% by weight lecithin, between 2.5% and 3.5% by weight isopropyl palmitate and between 1.5% and 2.5% by weight urea, preferably about 11.86%, 3.13%, 3.13% and 1.90% by weight, respectively, and between 76.5% to 83.5% by weight of further pharmaceutically acceptable excipients altogether.

One embodiment of the invention is the pharmaceutical composition according to the invention, wherein the pharmaceutically acceptable carrier comprised by the pharmaceutical composition comprises between 2.8% and 3.8% by weight lecithin, between 5.0% and 7.0% by weight isopropyl palmitate, between 0.5% and 2.0% by weight Polaxamer 407 and between 1.5% and 2.5% by weight urea, preferably about 3.3%, 6.0%, 1.0% and 2.0% by weight, respectively, and between 84.7% to 90.2% by weight of further pharmaceutically acceptable excipients altogether

One embodiment of the invention is a pharmaceutical composition according to the invention, wherein the pH of the composition is between 4.0 and 6.0, preferably between 4.5 and 5.5.

Despite what common general knowledge and the prior art may suggest, the pH of between 4.0 and 6.0 of the pharmaceutical composition of the invention effectively contributes to the efficacy of the vasoactive prostaglandin with regard to skin penetration, lack of side effects and time to an erection sufficient for sexual activity. The inventors surprisingly found that a pH of 7 or higher, *e.g.* a pH of 9, is not essential, in order to arrive at an efficacy of the therapy of over 90% in ED patients treated with the pharmaceutical composition of the invention. To the contrary, a pH of between 4.5 and 6.0, *e.g.* a pH of 5.3, proved to be beneficial for arriving at an efficacy of therapy of over 90%, *e.g.* 94%. For details, see the outcome of a clinical trial with the pharmaceutical composition of the invention, outlined in Example 3, below. Such a high efficacy of over 90% has not been reported for ED patients treated with any of the formulations referred to above (See summaries of references in 'Background of the invention').

Of course, creams comprising pharmaceutically acceptable excipients for emulsifying the cream and comprising pharmaceutically acceptable excipients for preserving the cream are known in the art, as is the application of such a cream as a pharmaceutically acceptable carrier. In fact, many creams applicable as pharmaceutically acceptable carrier are applicable for the pharmaceutical composition of the invention. According to the invention, any cream having a viscosity at 25°C of between 25.000 to 200.000 mPa.s, preferably between 40.000 to 150.000 mPa.s, more preferably between 50.000 to 100.000 mPa.s, and said cream comprising skin penetration enhancing compounds isopropyl myristate, lecithin, isopropyl palmitate and urea, or said cream comprising skin penetration enhancing compounds Polaxamer 407, lecithin, isopropyl palmitate and urea, is applicable for the pharmaceutical composition of the invention, wherein the cream preferably further comprises pharmaceutically acceptable excipients for emulsifying the cream and comprising pharmaceutically acceptable excipients for preserving the cream.

One embodiment of the invention is the pharmaceutical composition according to the invention, wherein the pharmaceutically acceptable carrier comprises:
i. water;
ii. glyceryl stearate;
iii. stearic acid;
iv. polyethylene glycol 40 stearate;
v. poly (dimethylsiloxane)-silicon dioxide, preferably simethicone;
vi. cetylalcohol;
vii. stearylalcohol;
viii. potassium sorbate;
ix. ethylenediaminetetraacetic acid;
x. benzoic acid;
xi. sorbic acid; and
xii. butylated hydroxytoluene.

According to the invention, water, glyceryl stearate, stearic acid, polyethylene glycol 40 stearate, poly (dimethylsiloxane)-silicon dioxide, preferably simethicone, cetylalcohol, and stearylalcohol, together provide for an efficient emulsification of the cream and the vasoactive prostaglandin.

One embodiment of the invention is the pharmaceutical composition according to the invention, wherein the pharmaceutically acceptable carrier comprises high-molecular cross-linked polymer of acrylic acid, preferably Carbomer 980 NF Polymer.

According to the invention, potassium sorbate, ethylenediaminetetraacetic acid, benzoic acid, sorbic acid, and butylated hydroxytoluene, together provide for an efficient preservation of the cream and the vasoactive prostaglandin, provided that the pharmaceutical composition of the invention is kept at a temperature of between 2°C and 8°C.

Preferably, the pharmaceutically acceptable carrier for the pharmaceutical composition of the invention is a cream, preferably *Pentravan* cream (Fagron), consisting of:
11.86% by weight isopropyl myristate;
3.13% by weight lecithin;
3.13% by weight isopropyl palmitate;
1.90% by weight urea;
61.78% by weight water;
4.75% by weight glyceryl stearate;
4.75% by weight stearic acid;
4.75% by weight PEG 40 stearate;
1.90% by weight simethicone;
0.95% by weight cetylalcohol;
0.48% by weight stearylalcohol;
0.19% by weight potassium sorbate;
0.10% by weight EDTA;
0.10% by weight benzoic acid;
0.10% by weight sorbic acid;
0.10% by weight butylated hydroxytoluene;
0.09% by weight Carbomer 980 NF Polymer,
thus comprising compounds enhancing skin penetration of a vasoactive prostaglandin according to the invention. Such Pentravan cream according to the invention has a viscosity ofbetween 40.000 mPa.s and 150.000 mPa.s.

An alternative embodiment of the invention is the pharmaceutical composition according to the invention, wherein the pharmaceutically acceptable carrier is *Cream-II* (Fagron) and wherein Cream-11 comprises:
Cetyl alcohol 1.75% by weight;
Stearyl alcohol 0.8% by weight;
Glyceryl monostearate 5.0% by weight;
Simethicone emulsion 30% USP (DC-7-9245 30%) 2.0% by weight;
Stearin (triple-pressed stearic acid) 4.0% by weight;
Sorbic acid 0.2% by weight;
Benzoic acid 0.1% by weight;
Isopropyl palmitate 6.0% by weight;
BHT (Butylated hydroxytoluene) 0.1% by weight;
Soy lecithin granulated 3.3% by weight;
Alpha tocopherol 0.1% by weight;
Urea 2.0% by weight;
Potassium sorbate 0.2% by weight;
PEG-40 Stearate (Lipopeg 39-S) 2.0% by weight;
Xanthan Gum 0.15% by weight;
Ethoxydiglycol 2.5% by weight;
EDTA-Na₂ 0.1% by weight;
Purified Water 62.2% by weight;
Aminomethylpropanol (AMP-95) 50% solution 2.5% by weight;
Polaxamer 407 20% (w/v) aqueous solution 5.0% by weight; and
Citric acid 20% (w/v) solution qs pH 5.5-6.0,
thus comprising compounds enhancing skin penetration of a vasoactive prostaglandin according to the invention.

Such cream according to the invention has a viscosity of between 40.000 mPa.s and 200.000 mPa.s.

Preferably, the pH of such carrier cream is between about 4.5 and 6.0 according to the invention, although a pH of higher than 6.0 may be equally suitable, for arriving at treatment efficacies of over 90%, *e.g.* 94%, when ED patients are treated with the pharmaceutical composition of the invention. In one embodiment of the invention, the pharmaceutical composition of the invention comprises a cream, wherein the cream is one of the two above-mentioned creams having a pH of between 4.5 and 5.5 and having a viscosity of between 40.000 mPa.s and 150.000 mPa.s.

A second aspect of the invention relates to a method for preparing a pharmaceutical composition according to the invention, comprising the steps of:
a. providing a pharmaceutically acceptable carrier wherein the carrier is formulated as a cream with a viscosity at 25°C of between 25.000 to 200.000 mPa.s, preferably between 40.000 to 150.000 mPa.s, more preferably between 50.000 to 100.000 mPa.s, said carrier comprising:
   i. lecithin;
   ii. isopropyl palmitate; and
   iii. urea,
      and said carrier further comprising either isopropyl myristate or a polaxamer, preferably Polaxamer 407;
b. providing a vasoactive prostaglandin, preferably alprostadil, and a solvent;
c. dissolving said prostaglandin in the solvent; and
d. mixing the dissolved vasoactive prostaglandin with said carrier,
wherein the pH of the carrier is between 4.0 and 6.0, preferably between 4.5 and 5.5, and the pharmaceutical composition is refrigerated to between about 2°C to about 8°C

One embodiment of the invention is the method for preparing a pharmaceutical composition according to the invention, wherein in step b. the solvent is 2-(2-ethoxyethoxy)ethanol, preferably the solvent is Transcutol P.

One embodiment of the invention is the method for preparing a pharmaceutical composition according to the invention, wherein in step c. the vasoactive prostaglandin is dissolved at a final concentration of between 1% and 10% by weight, preferably about 2,5% by weight in the solvent.

One embodiment of the invention is the method for preparing a pharmaceutical composition according to the invention, wherein in step c. the vasoactive prostaglandin is dissolved at a final concentration of between 1% and 10% by weight, preferably about 2,5% by weight in the solvent, and wherein the vasoactive prostaglandin is alprostadil and the solvent is Transcutol P.

One embodiment of the invention is the method for preparing a pharmaceutical composition according to the invention, wherein in step d. the dissolved vasoactive prostaglandin is mixed with the carrier up to a final ratio of between 1% to 30% by weight, preferably about 20% by weight.

One embodiment of the invention is the method for preparing a pharmaceutical composition according to the invention, wherein in step c. alprostadil is dissolved at a final concentration of about 2,5% by weight in Transcutol P, and wherein in step d. the dissolved alprostadil is mixed with the carrier up to a final ratio of about 20% by weight, such that the alprostadil concentration in the pharmaceutical composition is about 0,5% by weight.

The pharmaceutical composition provided by the method of the invention is ready-to-use, according to the invention. That is to say, the pharmaceutical composition is ready for use in a treatment regime for an ED patient, without any further preparations required. The inventors found that the selection of the cream with the indicated viscosity and having the indicated pH and comprising the compounds enhancing skin penetration of the pharmaceutically active ingredient according to the method of the invention, provides for a very stable pharmaceutical composition of the invention, when kept at between about 2°C to about 8°C.

Preferably, the viscosity of the carrier cream is between about 50.000 mPa.s and 70.000 mPa.s, more preferably, about 60.000 to 65.000 mPa.s in the method of the invention. Preferably, the pH of the carrier cream is about 5.0, for example about 5.3. Preferably, the carrier cream is Pentravan (Fagron, The Netherlands; pharmaceutically acceptable carrier I, see Examples, below) or the carrier cream is Cream-II (Fagron), as outlined above and comprising the indicated compounds enhancing skin penetration according to the invention, of an active pharmaceutical ingredient, *i.e*. vasoactive prostaglandin, according to the invention. Preferably, the vasoactive prostaglandin is alprostadil. Of course, as said before, according to the invention other carrier creams comprising the compounds enhancing skin penetration of a vasoactive prostaglandin according to the invention and having a viscosity in the range of 25.000 mPa.s and 200.000 mPa.s, preferably 40.000 mPa.s and 150.000 mPa.s, are equally suitable for use in the method of the invention.

A third aspect of the invention relates to a pharmaceutical composition of the invention or provided by the method according to the invention or obtainable by the method according to the invention for use in the treatment of erectile dysfunction.

Surprisingly, the inventors found that treatment of ED patients with the pharmaceutical composition according to the invention resulted in an unprecedented high efficacy of therapy. That is to say, out of a group of 64 ED patients, sixty patients (94%) presented efficient clinical response and 60 patients (94%) were satisfied with the outcome of the application of the pharmaceutical composition of the invention. See also Example 1-3, below.

Current therapies comprising a pharmaceutical composition comprising a vasoactive PGE1 such as alprostadil all have their drawbacks with regard to side effects, efficacy of the therapy and ease of use. For example, solid and hard tablets to be released from a stem 3 to 7 in length which first has to be inserted in the penis urethra, induce pain and can cause bleeding events. Furthermore, micturition is required before the tablet is to be positioned in the urethra. These aspects not only induce inconvenience due to the side effects, though also interfere with the social aspects before and during sexual activity. As a different example, pharmaceutical compositions comprising a vasoactive PGE1 such as alprostadil, which have to be administered by providing drops of the composition at or in the meatus of the glans penis, come with the drawback of the necessity to precisely direct the drops at the desired location. Furthermore, apart from relatively high rates of reported side effects, efficiency and efficacy of the various current therapies is mediocre at best.

Now, the inventors not only found a pharmaceutical composition comprising a vasoactive PGE1 such as alprostadil according to the invention, that provides the high efficacy of therapy, as mentioned before. In addition, the inventors also combined the potent ED treatment with a route of administration that is extremely convenient to the patient and efficiently facilitates application of the pharmaceutical composition at the desired site, *i.e*. the rim of the meatus of the glans of the penis and the fossa navicularis.

One embodiment of the invention is a pharmaceutical composition for use in the treatment of erectile dysfunction according to the invention, wherein for said use the composition is administered at the rim of the meatus of the glans penis and into the fossa navicularis. According to the invention, part of the pharmaceutical composition is deposited at the rim of the meatus, at the skin of the glans penis, and in addition part of the pharmaceutical composition is deposited in the fossa navicularis upon administration of the pharmaceutical composition for use in the treatment of erectile dysfunction according to the invention.

One embodiment of the invention is the use of the pharmaceutical composition of the invention or obtainable by the method of the invention for the manufacture of a medicament.

One embodiment of the invention is the use of the pharmaceutical composition of the invention or obtainable by the method of the invention for the manufacture of a medicament, wherein the medicament is for the treatment of erectile dysfunction.

One embodiment of the invention is the use of the pharmaceutical composition of the invention or obtainable by the method of the invention for the manufacture of a medicament, wherein the medicament is administered at the rim of the meatus of the glans penis and into the fossa navicularis.

One embodiment of the invention is the use of the pharmaceutical composition of the invention or obtainable by the method of the invention for the manufacture of a medicament, wherein the medicament is for the treatment of erectile dysfunction, and wherein the medicament is administered at the rim of the meatus of the glans penis and into the fossa navicularis.

The viscosity of the carrier cream in the pharmaceutical composition of the invention is selected such that when a dosage of the composition is administered at the rim of the meatus of the glans penis and in the fossa navicularis of the penis, the composition at the rim adheres to the skin of the glans and substantially remains at the site of administration without spilling composition, whereas the composition introduced in the fossa navicularis also stays within this cavity for a sufficient period of time allowing uptake of the PGE1 through the skin. Moreover, while administering the pharmaceutical composition of the invention, viscosity is such that the composition easily and conveniently enters the fossa navicularis through the meatus, without requiring to put painful force at the meatus and the glans penis.

One embodiment of the invention is the pharmaceutical composition of the invention for use in the treatment of ED, wherein the part of the pharmaceutical composition that is delivered at the rim of the meatus of the glans penis is rubbed into the glans penis within a few minutes after administration of the pharmaceutical composition, *e.g*. within between 1 to 5 minutes, preferably rubbed into the skin of the glans penis for between 10 to 30 seconds, preferably for about 15 seconds.

One embodiment of the invention is the pharmaceutical composition of the invention for use in the treatment of ED, wherein the composition is ready-to-use and wherein the composition exerts its erection-inducing activity within several minutes, that is to say within 5-30 minutes, preferably within 5-15 minutes after administering the composition, more preferably within even within a shorter period of time, according to the invention.

ED patients responding by receiving an erection upon application of the pharmaceutical composition of the invention reported that the erection prolonged for between 5 and 80 minutes, in 84.4% of the cases. One embodiment of the invention is the pharmaceutical composition of the invention for use in the treatment of ED, wherein the erection lasts for at least 5 minutes, preferably for at least 10 minutes, more preferably for at least 20 minutes, most preferably for between about 25 minutes to 1.5 hours. Preferably, the erection is prolonged for between 10 minutes and 80 minutes, more preferably between 10 and 30 minutes, for allowing to conduct satisfactory sexual activity.

Importantly, the inventors surprisingly found that upon application of the pharmaceutical composition of the invention, the ED patients to whom the pharmaceutical composition was administered built up an erection without the necessity of any further stimulus (See Examples, below). One embodiment of the invention is the pharmaceutical composition of the invention for use in the treatment of ED, wherein after administration of said composition to an ED patient the erection is established without stimulus, such as any visual or physical sexual stimulus known in the art. This aspect contributes to the ease and convenience for the ED patients to whom the pharmaceutical composition of the invention is administered, since the efficacy and efficiency of the pharmaceutical composition of the invention are autonomously established upon administration of the composition. The effect of the pharmaceutical composition of the invention is not dependent on any intervention required by the ED patient. This makes the pharmaceutical composition of the invention a surprisingly easy and reliable and convenient tool in solving the medical problem of the ED patient.

A fourth aspect of the invention relates to a ready-to-use applicator comprising the pharmaceutical composition of the invention or provided by the method according to the invention or obtainable by the method according to the invention, wherein the applicator comprises a flexible tip, preferably a flexible silicon tip, with an approximate diameter of between 0.30 cm and 1.00 cm, preferably between 0.50 cm and 0.65 cm, more preferably between 0.55 cm and 0.59 cm, and wherein the applicator contains between 1 and 250 unit doses of the vasoactive prostaglandin, preferably between 2 and 150 unit doses, more preferably between 3 and 100 unit doses.

As said, the pharmaceutical composition of the invention is ready-to-use with regard to administering the composition to an ED patient having the desire to present with an erection for the benefit of sexual activity. Therefore, the pharmaceutical composition of the invention is provided in an applicator which in itself is also ready-to-use. For example, the ready-to-use applicator is an *Airless Applicator-* pump applicator (Emphasys, BR), having a soft silicon tip with a diameter of 0.57 cm +/- 0.02 cm. When the ED patient is in need of an erection, the ready-to-use applicator comprising the ready-to-use pharmaceutical composition of the invention, is at his disposal for immediate use, according to the invention.

Applicators comprise 1 or several unit doses of the pharmaceutical composition of the invention, preferably 1 to 3 unit doses, or more than 3 unit doses. One embodiment of the invention is a ready-to-use applicator comprising the pharmaceutical composition of the invention, wherein the applicator comprises 1 to 3 unit doses of said composition, or a multiple of 2 or 3 unit doses, preferably about 100 unit doses. Preferably, a unit dose of the vasoactive prostaglandin, preferably alprostadil, in the pharmaceutical composition of the invention is comprised in between 0.010 to 0.040 gram of the composition, more preferably in about 0.020 gram. Preferably, a unit dose of the vasoactive prostaglandin in the pharmaceutical composition of the invention, preferably alprostadil, is between 50 and 500 microgram, more preferably, 100 or 200 or 300 microgram, most preferably, about 100 microgram. A preferred embodiment of the invention is the ready-to-use applicator comprising the pharmaceutical composition of the invention, wherein the vasoactive prostaglandin is alprostadil, and wherein the applicator comprises about 250 unit doses of about 100 microgram of the alprostadil, wherein each unit dose is comprised in about 0.020 gram of the composition, according to the invention.

The ready-to-use applicator according to the invention, comprising the pharmaceutical composition of the invention is a single-use applicator, preferably a multiple-use applicator. The single-use applicator of the invention preferably contains between 1 to 5 unit doses of the vasoactive prostaglandin, preferably alprostadil, preferably between 1 to 3 unit doses. The multiple-use applicator of the invention preferably contains between 30 to 250 unit doses preferably each of about 0.020 gram, preferably comprising about 100 microgram alprostadil. According to the invention, pharmaceutical composition remaining in the ready-to-use multiple-use applicator, after use of the applicator, is stored again refrigerated, preferably at between 2°C and 8°C.

One embodiment of the invention is the pharmaceutical composition for use in the treatment of erectile dysfunction according to the invention, wherein said pharmaceutical composition is administered with the ready-to-use applicator of the invention.

One embodiment of the invention is a ready-to-use applicator comprising the pharmaceutical composition according to the invention for use in the treatment of erectile dysfunction according to the invention, wherein said applicator is adapted for positioning at the rim and above the meatus of the glans penis and wherein administration of said composition is at the rim of the meatus and into the fossa navicularis.

One embodiment of the invention is the use of the pharmaceutical composition of the invention or obtainable by the method of the invention for the manufacture of a medicament, wherein the medicament is administered with the ready-to-use applicator of the invention.

The ready-to-use applicator comprising the pharmaceutical composition of the invention provides numerous benefits in view of current applicators comprising current pharmaceutical compositions comprising a vasoactive prostaglandin. The soft and flexible tip, *e.g.* a silicon tip, allows for convenient and painless adaptation of the applicator to the rim of the meatus. The dimension of the flexible tip, *i.e*. the tip having a diameter of between 0.30 cm and 1.00 cm, preferably about 0.57 cm, is such that the tip is easily positioned at the rim of the meatus whereby the opening of the tip is simultaneously positioned above the meatus. Herewith, dispensing pharmaceutical composition of the invention with the applicator allows for precise and efficient administration of the composition both in part at the rim of the meatus and in part inside the fossa navicularis.

One embodiment of the invention is the pharmaceutical composition for use in the treatment of erectile dysfunction according to the invention, wherein said pharmaceutical composition is administered with the ready-to-use applicator of the invention and wherein the administered dose of the vasoactive prostaglandin is between 50 to 500 microgram, preferably between 100 to 300 microgram, more preferably about 100 microgram or about 200 microgram or about 300 microgram, wherein the vasoactive prostaglandin preferably is alprostadil.

A fifth aspect of the invention relates to a kit comprising:
a. the ready-to-use applicator comprising the pharmaceutical composition of the invention;
b. a container for storage of the applicator and for maintaining the temperature of the pharmaceutical composition in the applicator at or below a predetermined temperature for a predetermined period of time; and
c. instructions regarding storage and use of the applicator and use of the pharmaceutical composition.

One embodiment of the invention is a kit of the invention, wherein the container comprises one or more of the materials selected from polyamide filament, ethylene vinyl acetate, polystyrene, or any combination thereof, and wherein the predetermined temperature is set to between 2°C to 15°C, preferably between 2°C to 12°C, more preferably to about 8°C, and wherein the predetermined period of time is set to between 1 hour to 12 hours, preferably to between 3 hours to 8 hours, more preferably to about 4 hours, according to the invention.

A further aspect of the invention relates to a method of treatment of erectile dysfunction, wherein the pharmaceutical composition of the invention is administered to a patient in need thereof.

An aspect of the invention is the use of the pharmaceutical composition of the invention for the treatment of erectile dysfunction.

An aspect of the invention is a method of treating a patient suffering from erectile dysfunction comprising administering the pharmaceutical composition of the invention to the patient.

An aspect of the invention is the administration of the pharmaceutical composition of the invention for treating erectile dysfunction.

An aspect of the invention is the pharmaceutical composition of the invention for treating erectile dysfunction.

The present invention will be illustrated further by means of the following non-limiting Examples.

### EXAMPLES

### EXAMPLE 1

### Preparation of a pharmaceutically acceptable carrier

Batches of a pharmaceutically acceptable carrier I, referred to as 'Batch A' and 'Batch B', were prepared by mixing pharmaceutically acceptable excipients selected from excipients promoting skin permeation of API, excipients aiding in emulsifying the carrier, excipients for preservation of the carrier. The excipients promoting skin permeation of API are altogether also referred to as skin penetration enhancer system. The pharmaceutically acceptable carrier I is a cream and is commercially available under the trade name *Pentravan* (Fagron).

For Batch A and Batch B, the excipients promoting skin permeation of API were isopropyl myristate, lecithin, isopropyl palmitate and urea; the excipients aiding in emulsifying the carrier were water, glyceryl stearate, stearic acid, PEG 40 stearate, the poly (dimethylsiloxane)-silicon dioxide simethicone, cetylalcohol, stearylalcohol, the high-molecular cross-linked polymer of acrylic acid Carbomer 980 NF Polymer; and the excipients for preservation of the carrier were potassium sorbate, EDTA, benzoic acid, sorbic acid, butylated hydroxytoluene. The following amounts of pharmaceutically acceptable excipients were mixed for Batch A and Batch B:
11.86% by weight isopropyl myristate;
3.13% by weight lecithin;
3.13% by weight isopropyl palmitate;
1.90% by weight urea;
61.78% by weight water;
4.75% by weight glyceryl stearate;
4.75% by weight stearic acid;
4.75% by weight PEG 40 stearate;
1.90% by weight simethicone;
0.95% by weight cetylalcohol;
0.48% by weight stearylalcohol;
0.19% by weight potassium sorbate;
0.10% by weight EDTA;
0.10% by weight benzoic acid;
0.10% by weight sorbic acid;
0.10% by weight butylated hydroxytoluene;
0.09% by weight Carbomer 980 NF Polymer.

The pharmaceutically acceptable carrier I is a medium viscous cream having a density of between 0.97 to 1.00 g/ml. The viscosity is between 40.000 to 150.000 mPa.s. The pH is between 4.5 and 5.5.

For Batch A, the density was 0.98 g/ml. Viscosity at 25°C was 61.348 mPa.s. PH was 5.3. Values for Batch B were similar.

The pharmaceutically acceptable carrier I as described was specially designed for enabling prostaglandin in the pharmaceutical composition comprising vasoactive prostaglandin and a pharmaceutically acceptable carrier according to the invention, to reach the main action receptor in the corpus cavernosum penis. With regard to the efficiency and efficacy of the cream, the excipients promoting skin permeation were essential in the pharmaceutically acceptable carrier I.

An alternative combination of excipients promoting skin permeation in the pharmaceutically acceptable carrier according to the invention comprises lecithin, isopropyl palmitate, urea and a polaxamer, preferably Polaxamer 407, wherein isopropyl myristate is omitted in the carrier.

Alternative excipients aiding in emulsifying the carrier in the pharmaceutically acceptable carrier according to the invention comprise a thickener such as xanthan gum, which is more stable than thickener Carbomer 980 NF Polymer of pharmaceutically acceptable carrier I according to the invention, for further improvement of the stability of the cream. Xanthan gum is a polysaccharide secreted by the bacterium *Xanthomonas campestris*, known in the art.

Alternative excipients aiding in emulsifying the carrier in the pharmaceutically acceptable carrier according to the invention comprise anti-oxidant alpha tocoferol in the preservative system of the cream.

The pharmaceutically acceptable carrier I has been proven to efficiently aid the percutaneous absorption of *e.g.* hormones, showing the beneficial permeation performance of the transdermal vehicle cream [Polonini *et al.* (2014)].

A second pharmaceutically acceptable carrier II was prepared similarly, and a Batch C consisted of:
Cetyl alcohol 1.75% by weight;
Stearyl alcohol 0.8% by weight;
Glyceryl monostearate 5.0% by weight;
Simethicone emulsion 30% USP (DC-7-9245 30%) 2.0% by weight;
Stearin (triple-pressed stearic acid) 4.0% by weight;
Sorbic acid 0.2% by weight;
Benzoic acid 0.1% by weight;
Isopropyl palmitate 6.0% by weight;
BHT (Butylated hydroxytoluene) 0.1% by weight;
Soy lecithin granulated 3.3% by weight;
Alpha tocopherol 0.1% by weight;
Urea 2.0% by weight;
Potassium sorbate 0.2% by weight;
PEG-40 Stearate (Lipopeg 39-S) 2.0% by weight;
Xanthan Gum 0.15% by weight;
Ethoxydiglycol 2.5% by weight;
EDTA-Na₂ 0.1% by weight;
Purified Water 62.2% by weight;
Aminomethylpropanol (AMP-95) 50% solution 2.5% by weight;
Polaxamer 407 20% (w/v) aqueous solution 5.0% by weight; and
Citric acid 20% (w/v) solution qs pH 5.5-6.0.

The excipients promoting permeation of an API through the skin, comprised in pharmaceutically acceptable carrier II, consist of isopropyl palmitate (penetration enhancer), soy lecithin (penetration enhancer), ethoxydiglycol (penetration enhancer), urea (penetration enhancer) and Polaxamer 407 (tensoactive penetration enhancer).

Batches of pharmaceutically acceptable carrier were tested for their microbial load. The total aerobic microbial count (TAMC) was determined, as well as the total yeast and mold count (TYMC), the load of *Pseudomonas aeruginosa* and the load of *Staphylococcus aureus.* TAMC should be at or below 1.000 CFU/g, TYMC should be at or below 100 CFU/g.

For example, for Batch A of pharmaceutically acceptable carrier I, the microbial load was tested and was within the aforementioned specifications.

### EXAMPLE 2

### Preparation of a pharmaceutical composition comprising vasoactive prostaglandin and a pharmaceutically acceptable carrier

The pharmaceutical composition comprising vasoactive prostaglandin and a pharmaceutically acceptable carrier according to the invention was prepared by mixing alprostadil dissolved in Transcutol P with Batch A of pharmaceutically acceptable carrier I (see above). Alprostadil was purchased from Shanghai Bioman Pharma Limited (China).

Alprostadil is the synthetic form of natural prostaglandin E1. The molecular formula is C₂₀H₃₄O₅ and the IUPAC name is *7*-[(1*R*,*2R*,*3R*)-*3*-*hydroxy*-*2*-[(*3S*)-*3*-*hydroxyoct*-1-*en*-1-*yl*]-*5-oxocyclopentyl]heptanoic acid* or *7-[(1R,2R,3R)-3-hydroxy-2-[(E,3S)-3-hydroxyoct-1-enyl]-5-oxocyclopentyl]heptanoic acid.*

Ten mg of alprostadil was solubilized in the solvent 2-(2-ethoxyethoxy)ethanol Transcutol P; Transcutol P was added to the alprostadil up to a final solution 400 mg in weight. The 400 mg of completely solubilized alprostadil in Transcutol P (comprising 10 mg alprostadil) was added to 1600 mg of Batch A of pharmaceutically acceptable carrier I, and the cream was completely homogenized. The pharmaceutical formulation is kept refrigerated at between 2°C and 8°C. Alprostadil concentration: 100 microgram per 20 mg of the pharmaceutical formulation. Viscosity of the pharmaceutical composition was similar to the viscosity of the cream.

*Airless Applicators* pump applicator (Emphasys, BR) were filled with the pharmaceutical formulation. The applicators comprised a flexible silicon tip with an external diameter of 5.70 mm +/- 0.20 mm and an outlet of the tip with a diameter of 0.70 mm +/- 0.05 mm. See Fig. 1B. One pump with the applicator dispensed 20 mg of the pharmaceutical formulation, comprising 100 microgram alprostadil. An applicator had a capacity of 5 gram, having dimensions of 126 mm times 17 mm.

Applicators were individually packed in a thermal case having dimensions of 17 cm times 4 cm times 4 cm. The case was a packing set thermic framed in ethylene vinyl acetate, coated in the polyamide filament material helanca, with a double cradle in the closed-cell extruded polystyrene foam *Styrofoam*. The thermal case kept the filled applicator at a temperature at or below 8°C for at least 4 hours (thermal case placed at ambient temperature).

The thermal case comprising an applicator filled with the pharmaceutical formulation was supplied with instructions on storage and use of the applicator and the pharmaceutical composition of the invention, according to the invention.

Alternatively, similar to the pharmaceutical formulation described above, the 400 mg of completely solubilized alprostadil in solvent (comprising 10 mg alprostadil) was added to 1600 mg of Batch C of pharmaceutically acceptable carrier II, and the cream was completely homogenized.

### EXAMPLE 3

### Use of the pharmaceutical composition comprising vasoactive prostaglandin and a pharmaceutically acceptable carrier according to the invention, in the treatment of erectile dysfunction

The pharmaceutical composition comprising vasoactive prostaglandin and a pharmaceutically acceptable carrier according to the invention was tested for its efficacy in a clinical trial with 64 human male patients included.

The tested pharmaceutical composition was the composition according to the invention, prepared as outlined in Example 2, wherein the 400 mg of completely solubilized alprostadil in solvent (comprising 10 mg alprostadil) was added to 1600 mg of Batch A of pharmaceutically acceptable carrier I, followed by complete homogenization of the cream.

### Trial participants

All patients presented themselves spontaneously to urological medical appointment with erectile dysfunction as main complaint or erectile dysfunction previous diagnosis or previous erectile penile prosthesis use.

Number of patients (n): 64. Age from 27 to 73 years old.
38 patients with hypertension
12 patients with diabetes
2 patients with Peyronie disease
4 patients with premature ejaculation
5 patients with ADAM
3 patients with prosthesis penile (cold glans syndrome)

### Methodology

All patients were subjected to the pharmaceutical composition of the invention (see Example 2 for details; pharmaceutically acceptable carrier I) using the ready-to-use applicator comprising alprostadil in cream (200 microgram per dose according to the invention; 2 pumps of the applicator per occasion). Patients were provided with instructions with regard to the use of the read-to-use applicator comprising the pharmaceutical composition of the invention.

The instructions in brief: The ready-to-use applicator had to be kept cool up to just prior to use. Patients were instructed to expose the glans (head of the penis), hold it between fingers promoting opening of the urethral meatus hole (tip of the penis). With their other hand, ED patients had to held the applicator with the silicon tip at the orifice and to press the embolus until to the end so that the pharmaceutical composition (here, alprostadil in Pentravan cream) was applied inside the urethral meatus (penis orifice) and in its surroundings, between 5 to 15 minutes before intercourse. Upon administering the pharmaceutical composition, the composition partly remains on the skin of the glans penis and partly enters the fossa navicularis. The part remaining at the glans penis had to be massaged gently until the pharmaceutical composition was absorbed (taking on average 15 seconds of rubbing). The applicator with the remaining pharmaceutical composition had to be stored at 2°C to 8°C after administering the composition.

### Results

### General results

Sixty patients (93,75%) presented efficient clinical response and 4 of them did not present response: one of the non-responders, 39 years old with premature ejaculation, 2 of the non-responders with IV grade hypertension and insulin dependent diabetes and one non-responder in late post-operative of removal of penile prosthesis process due to infection.

Tree of the patients with penile prosthesis with cold glans syndrome presented satisfactory or very satisfactory clinical response.

Penetration capacity using OATES classification:
Grade 0 - 4 patients
Grade 1 - 7 patients
Grade 2 - 11 patients
Grade 3 - 42 patients

Prolonged erection (priapism) - no patient
60 to 80 minutes erection - 2 patients
30 to 60 minutes erection - 5 patients
10 to 30 minutes erection - 31 patients
05 to 10 minutes erection - 16 patients
< 05 minutes erection - 6 patients
no erection - 4 patients (*i. e.* patients with no clinical response)

### Adverse reactions

Glans hyperemia - 13 patients
Dysuria - 1 patient
Urethral burning - 3 patients
Changes in heart frequency (not more than 15% compared before test) - 3 patients
Elevated blood pressure more than 20% from baseline - 2 patients
Elevated blood pressure between 15% to 20% from baseline - 1 patient
Elevated blood pressure between 10% to 15% from baseline - 3 patients
Elevated blood pressure between 05% to 10% from baseline - 1 patient
Headache (not associated with elevated blood pressure or heart frequency) - 2 patients

### Additional observations

Fear of application if alone - 1 patient

### Satisfaction with the product

56 patients declared satisfied or very satisfied.
4 patients declared little satisfied and 4 patients were dissatisfied.

### Conclusion

Sixty patients (93,75%) presented efficient clinical response and 60 patients (93,75%) were satisfied.

### EXAMPLE 4

### Preservation of the pharmaceutical composition

Applicators filled with pharmaceutical composition according to the invention were cooled to a temperature between 2°C and 8°C. Applicators were *Airless Applicator-* pump applicators (Emphasys, BR). The applicators were then individually packed in a thermal case ('cooling case') having dimensions of 17 cm times 4 cm times 4 cm. The case was a packing set thermic framed in ethylene vinyl acetate, coated in the polyamide filament material *helanca,* with a double cradle in the closed-cell extruded polystyrene foam *Styrofoam*. The thermal case kept the pre-cooled filled applicators at a temperature between 2°C and 8°C for 4 hours (thermal case placed at ambient temperature).

### EXAMPLE 5

### Skin penetration of testosterone in pharmaceutically acceptable carrier

Batches of pharmaceutically acceptable carrier I (Pentravan; see above) and pharmaceutically acceptable carrier II (see above) were supplied with testosterone, to a final testosterone concentration of 50 milligram per gram of the cream. Of the pharmaceutically acceptable carrier I and II with testosterone, 8.40 mg was applied on dermatomed human skin (male, 30 years of age). The dermatomed skin sample had a thickness of 500 micrometer. Thus, 0.42 mg testosterone was applied onto the skin with the creams, *i.e*. 0.27 microgram per cm² skin. Testosterone absorption was followed in time at t = 1 h, 2 h, 4 h, 8 h, 12 h and 24 h. Permeation of testosterone through the skin was profiled. Data was gathered using HPLC and HPLC chromatograms were analyzed with regard to amount of testosterone present in an HPLC sample.

For the pharmaceutically acceptable carrier I (Pentravan), 57.3% of the testosterone applied to the skin was absorbed by the skin. For the pharmaceutically acceptable carrier II, 69.4% of the testosterone applied to the skin was absorbed by the skin.

These data demonstrate the applicability and the efficiency of the two pharmaceutically acceptable carriers with regard to facilitating the transfer of an active pharmaceutical ingredient through the human skin. Furthermore, these data demonstrate that both carriers have similar efficiency with regard to facilitating skin penetration of the compound.

Based on these results and based on the clinical trial data obtained with the pharmaceutical composition comprising vasoactive prostaglandin and the pharmaceutically acceptable carrier I according to the invention (outlined in Example 3), pharmaceutically acceptable carrier I and II are equally suitable for use in the pharmaceutical composition comprising vasoactive prostaglandin and a pharmaceutically acceptable carrier according to the invention.

### EXAMPLE 6

### Viscosity of the pharmaceutical compositions

Viscosity of pharmaceutically acceptable carrier I (Pentravan; Fagron B.V., The Netherlands) at 25°C is between 40.000 mPa.s and 150.000 mPa.s and preferably between about 50.000 mPa.s to 80.000 mPa.s.

The pharmaceutical composition according to the invention has substantially the same viscosity as the pharmaceutically acceptable carrier comprised in the pharmaceutical composition.

The viscosity of pharmaceutically acceptable carrier I comprising 20% by weight solvent, *i.e*. Transcutol P, was determined at 20,5°C and at 4°C. The pharmaceutically acceptable carrier with the indicated amount of solvent reflects the composition of the pharmaceutical composition outlined in Example 2, as used for the clinical trial outlined in Example 3.

### Method

The viscosity measurement was carried out in a rotational viscosimeter (Fungilab; model Visco Basic Plus), equipped with a Heldal Unit (used for determining viscosity of substances that do not flow). Spindle PB at 4 rpm was used for the determining of viscosity at room temperature and Spindle PD at 2 rpm for the determination of viscosity at 4°C. Samples of the pharmaceutically acceptable carrier I comprising 20% by weight Transcutol P were preserved in tight closed vases and protected from direct light, before measurements.

### Results

The viscosity of pharmaceutically acceptable carrier I comprising 20% by weight Transcutol P was 70.940,0 mPa.s at 20,5°C (ambient temperature). The viscosity of pharmaceutically acceptable carrier I comprising 20% by weight Transcutol P was 676.550,0 mPa.s at 4°C.

### REFERENCES

- H.C. Polonini, M.A.F. Brandao, A.O Ferreira, C. Ramos, N.R.B. Raposo, Evaluation of percutaneous absorption performance for human female sexual steroids into Pentravan cream, International Journal of Pharmaceutical Compounding, Vol. 18 No. 4, July-August 2014, pp. 332-340.
- Moncada & B. Cuzin, Clinical efficacy and safety of Vitaros©/Vivirec© (Alprostadil cream) for the treatment of erectile dysfunction, Urologia, 2015, published online at 3 March 2015.
- K. Moisidis, N. Kalinderis, K. Hatzimouratidis, Current role of local treatments for erectile dysfunction in the real-life setting, Current Opinion in Urology, 2016, Vol.26, pp. 123-128.
- H. Padma-Nathan & J.L. Yeager, An integrated analysis of alprostadil topical cream for the treatment of erectile dysfunction in 1732 patients, Journal of Urology, 2006 Vol. 68, pp. 386-391.
- E. Becher, Topical alprostadil cream for the treatment of erectile dysfunction, Expert Opinion on Pharmacotherapy, 2004 Vol. 5 No. 3, pp. 623-632.
- P. Costa & A.-J. Potempa, Intraurethral alprostadil for erectile dysfunction, Drugs, 2012 Vol. 72 No. 17, pp. 2243-2254.
- Saleh, H. Abboudi, M.B. Ghazal-Aswad, E.K. Mayer, J.A. Vale, Management of erectile dysfunction post-radical prostatectomy, Research and Reports in Urology 2015 Vol. 7, pp. 19-22.
- S. Leungwattanakij, V. Flynn, W.J.G. Hellstrom, Intracavernosal injection and intraurethral therapy for erectile dysfunction, Urologic Clinics of North America, May 2001, Vol. 28 No. 2, pp. 343-354.
- Datasheet for Polaxamer 407, sold under the tradename *Kolliphor P 407* by BASF, dated 24 April 2013.
- Datasheet for Carbopol 980 NF Polymer, of 16 January 2015, Lubrizol (BE).
- Handling sheet TRANSCUTOL P, Gattefossé, Saint Priest, France, dated 29 June 2010.
- Technical Data sheet TRANSCUTOL P, specification number 3260 / 4, dated 22 September 2008, Gattefossé, Saint Priest, France.
- ANGUS Chemie GmbH Technical Data Sheet AMP-95, CAS Registry No. 124-68-5, ANGUS Chemie GmbH, Ibbenbüren, DE.
- Safety Data Sheet, Ixom Operations Pty Ltd (Bronson & Jacobs division), Villawood, AU, dated 20 October 2015 - Version 3.

## Claims

1. Pharmaceutical composition comprising vasoactive prostaglandin and a pharmaceutically acceptable carrier, said carrier comprising:
a. lecithin;
b. isopropyl palmitate; and
c. urea,
wherein said carrier further comprises either isopropyl myristate or a polaxamer, preferably Polaxamer 407, and
wherein the carrier is formulated as a cream with a viscosity at 25°C of between 25.000 to 200.000 mPa.s.

2. Pharmaceutical composition according to claim 1, wherein the vasoactive prostaglandin is selected from natural prostaglandin E1, synthetic prostaglandin, preferably synthetic prostaglandin E1, more preferably alprostadil, a functional derivative of any of these prostaglandins, or any combination of these prostaglandins and/or a functional derivative thereof.

3. Pharmaceutical composition according to claim 2, wherein the vasoactive prostaglandin is alprostadil.

4. Pharmaceutical composition according to any of the claims 1 to 3, wherein the composition comprises between 0.3 and 1.0 % by weight vasoactive prostaglandin, preferably about 0.5% by weight.

5. Pharmaceutical composition according to any of the claims 1 to 4, wherein the pharmaceutically acceptable carrier comprises between 10% and 14% by weight isopropyl myristate, between 2.5% and 3.5% by weight lecithin, between 2.5% and 3.5% by weight isopropyl palmitate and between 1.5% and 2.5% by weight urea, preferably about 11.86%, 3.13%, 3.13% and 1.90% by weight, respectively, and between 76.5% to 83.5% by weight of further pharmaceutically acceptable excipients altogether.

6. Pharmaceutical composition according to any of the claims 1 to 4, wherein the pharmaceutically acceptable carrier comprises between 2.8% and 3.8% by weight lecithin, between 5.0% and 7.0% by weight isopropyl palmitate, between 0.5% and 2.0% by weight of a polaxamer, preferably Polaxamer 407 and between 1.5% and 2.5% by weight urea, preferably about 3.3%, 6.0%, 1.0% and 2.0% by weight, respectively, and between 84.7% to 90.2% by weight of further pharmaceutically acceptable excipients altogether.

7. Pharmaceutical composition according to any of the claims 1 to 6, wherein the pH of the composition is between 4.0 and 6.0, preferably between 4.5 and 5.5.

8. Pharmaceutical composition according to any of the claims 1 to 7, wherein the pharmaceutically acceptable carrier comprises:
i. water;
ii. glyceryl stearate;
iii. stearic acid;
iv. polyethylene glycol 40 stearate;
v. poly (dimethylsiloxane)-silicon dioxide, preferably simethicone;
vi. cetylalcohol;
vii. stearylalcohol;
viii. potassium sorbate;
ix. ethylenediaminetetraacetic acid;
x. benzoic acid;
xi. sorbic acid; and
xii. butylated hydroxytoluene.

9. Method for preparing a pharmaceutical composition of any of the claims 1 to 8, comprising the steps of:
a. providing a pharmaceutically acceptable carrier wherein the carrier is formulated as a cream with a viscosity at 25°C of between 25.000 to 200.000 mPa.s, preferably between 40.000 to 150.000 mPa.s, more preferably between 50.000 to 100.000 mPa.s, said carrier comprising:
i. lecithin;
ii. isopropyl palmitate; and
iii. urea,
and said carrier further comprising either isopropyl myristate or a polaxamer, preferably Polaxamer 407;
b. providing a vasoactive prostaglandin, preferably alprostadil, and a solvent;
c. dissolving said prostaglandin in the solvent; and
d. mixing the dissolved vasoactive prostaglandin with said carrier,
wherein the pH of the carrier is between 4.0 and 6.0, preferably between 4.5 and 5.5, and the pharmaceutical composition is refrigerated to between about 2°C to about 8°C.

10. Pharmaceutical composition of any of the claims 1 to 8 or obtainable by the method of claim 9 for use in the treatment of erectile dysfunction.

11. Pharmaceutical composition for use in the treatment of erectile dysfunction according to claim 10, wherein for said use the composition is administered at the rim of the meatus of the glans penis and into the fossa navicularis.

12. Ready-to-use applicator comprising the pharmaceutical composition of any of the claims 1 to 8 or obtainable by the method of claim 9, wherein the applicator comprises a flexible tip, preferably a flexible silicon tip, with an approximate diameter of between 0.30 cm and 1.00 cm, preferably between 0.50 cm and 0.65 cm, more preferably between 0.55 cm and 0.59 cm, and wherein the applicator contains between 1 and 250 unit doses of the vasoactive prostaglandin, preferably between 2 and 150 unit doses, more preferably between 3 and 100 unit doses.

13. Pharmaceutical composition for use in the treatment of erectile dysfunction according to claim 10 or 11, wherein said pharmaceutical composition is administered with the ready-to-use applicator of claim 12.

14. Kit comprising:
a. the ready-to-use applicator comprising the pharmaceutical composition of claim 12;
b. a container for storage of said applicator and for maintaining the temperature of said pharmaceutical composition in the applicator at or below a predetermined temperature for a predetermined period of time; and
c. instructions regarding storage and use of the applicator and use of the pharmaceutical composition.

15. Kit according to claim 14, wherein the container comprises one or more of the materials selected from polyamide filament, ethylene vinyl acetate, polystyrene, or any combination thereof, and wherein the predetermined temperature is set to between 2°C to 15°C, preferably between 2°C to 12°C, more preferably to about 8°C, and wherein the predetermined period of time is set to between 1 hour to 12 hours, preferably to between 3 hours to 8 hours, more preferably to about 4 hours.
